# EUROPEAN PATENT SPECIFICATION

(11) **EP 1 107 762 B1**
(45) Date of publication and mention of the grant of the patent: **26.10.2005**
(21) Application number: 99936893.9
(22) Date of filing: 13.08.1999
(51) Int. Cl.: A61K 31/557, A61P 15/00

(54) **USE OF MISOPROSTOL OR/AND MISOPROSTOL ACID FOR PREPARING DRUG IN ORDER TO CURE SEXUAL DYSFUNCTION IN WOMEN**
VERWENDUNG VON MISOPROSTOL ODER/UND MISOPROSTOL SAÜRE ZUR HERSTELLUNG EINES ARZNEIMITTELS ZUR BEHANDLUNG WEIBLICHER SEXUALER STÖRUNG
UTILISATION DE MISOPROSTOL ET/OU DE SON ACIDE POUR PREPARER UN PRODUIT PHARMACEUTIQUE SERVANT A SOIGNER LE DYSFONCTIONNEMENT SEXUEL CHEZ LA FEMME

(30) Priority: 14.08.1998 GR 98100315
(43) Date of publication of application: 20.06.2001
(73) Proprietor: Karouzakis, Petros, 106 77 Athens (GR); Kanakaris, Panagiotis, 106 77 Athens (GR)
(72) Inventor: Karouzakis, Petros, 106 77 Athens (GR); Kanakaris, Panagiotis, 106 77 Athens (GR)
(74) Representative: Froud, Clive
(86) International application number: PCT/GR1999/000030
(87) International publication number: WO 2000/009134

(56) References cited:
- DATABASE PASCAL [Online] INIST, CNRS (CENTRE NATIONAL DE LA RECHERCHE SCIENTIFIQUE), VANDOEUVRE-LES-NANCY, FR 96-0459457, 1996 MUNDLE ET AL: "vaginal misoprostol for induction of labor" XP002103712 & MUNDLE ET AL: "vaginal misoprostol for induction of labor" OBSTETRICS AND GYNECOLOGY, vol. 88, no. 4, 1996, pages 521-525,
- DATABASE MEDLINE [Online] 98376572, June 1998 (1998-06) CARBONELL ET AL: "vaginal misoprostol for early second-trimester abortion" XP002103713 & CARBONELL ET AL: "vaginal misoprostol for early second-trimester abortion" EUR J CONTRACEPT REPROD HEALTH CARE, vol. 3, no. 2, June 1998 (1998-06), pages 93-98,

## Description

The invention relates to the use of an already known pharmaceutical substance, misoprostol as well as its first metabolite, misoprostol acid, for the preparation of a drug for external use which is aimed at treating sexual dysfunction in women. By way of background, reference may be made, for example, to Mundle, W.R, and Young, D.C., "Vaginal misoprostol for induction of labour: a randomized controlled trial", Obstetrics & Gynecology, 88, No. 4 (Part 1), 521-525, October 1996, and to Carbonell, J.L, *et al*. "Vaginal misoprostol for early second-trimester abortion", European Journal of Contraception and Reproductive Health Care 1998; 3:93-98.

Even though the problem of female sexual dysfunction was recognized by modern medicine decades ago, it hasn't been confronted with efficiency yet. The magnitude of the problem is not quite known (Scrip Reports, March 1998), but according to an old report (Frank *et al*, 1978) the percentage of women facing a kind of sexual dysfunction reaches 63%.

In our days sexual dysfunction in women is being dealt with either with surgical reconstruction when (rarely) it is related to anatomic problems, or with psychotherapy, that could be effective in cases where the causes are not functional, or even with a special treatment involving hormone replacement in cases where sexual inability is related to hormonal disturbance.

These methods are being confronted with scepticism, either because they are applicable to a very small percentage of women (e.g. women with anatomic problems), or because they are characterized by relatively low efficiency, often in combination with a low benefit/risk ratio. Nowadays, the interest of many researchers is focused on the use of vasoactive substances, in accordance with the methods used in the treatment of male impotence. However, although these methods are successfully used in men (for example intracavemosal injections), they fail in women either because they strike against the female genital system anatomy (inability of self-injection into the corpora cavemosa of the clitoris), or because of the inefficiency of the methods currently available for external use.

The present invention aims at the removal of the disadvantages of the above methods and consists of the local application of a vasoactive substance, known as misoprostol, to the clitoris or/and to the vagina, in order to cure sexual dysfunction in women due to vascular, hormonal, phychogenic or other cause.

Misoprostol is the general name of a synthetic prostaglandin belonging to the E₁ series (PGE₁ analogs). Synthesis: P.W. Collins, R Pappo, Belgian Patent 827.127. American Patent 3,965,143 (The Merck Index, ed. Merck & Co. Inc., 11^{th} edition, 1989, p. 6128). Its chemical name is (11a,13E)-(±)-11,16-Dihydroxy-16-methyl-9-oxoprost-13-en-l-oic acid methyl ester or (±)-methyl-(1R,2R,3R)-3-hydroxy-2-[(E)-(4RS)-4-hydroxy-4-methyl-1-octenyl]-5-oxocyclopentaneheptanoate or (±)-15-deoxy-(16RS)-16-hydroxy-16-methyl-PGE₁ methyl ester. It consists of 4 stereoisomers in about equal proportions [(+)&(-) enantiomers of 16R- and 16S-forms], (The Merck Index, 11^{th} edition, 1989, p. 6128). The empirical formula is C₂₂H₃₈O₅. Its structural formula appears in page 8, Fig. 1.

Compared with other prostaglandins of Group E₁ and especially alprostadil, misoprostol bears a methyl group (-CH₃) on the carbon atom of position 16.

According to a method which relates the biological action of various medicament molecules to their chemical structure it appears that due to this group there exists a high penetration of misoprostol in the underlying tissues and a local vasodilation which treats sexual dysfunction. Misoprostol is used today orally as an anti-ulcer drug (Physicians Desc Reference, PDR, ed. Medical Economics Data, Production Company at Montrale 48^{th} edition, 1994, p. 2197-2199).

In particular, misoprostol is administered for the prevention of gastric ulcer to patients who take non-steroid antiinflammatory drugs. It is available in many European countries and USA by Searle Company under the commercial name Cytotec®. In no country is the drug mentioned as suitable for sexual dysfunction nor are there any relevant reports on the international bibliography. On the contrary, amongst the undesirable effects in oral therapy with misoprostol is male impotence (Physicians Desc Reference, ed. Medical Economics Data, Production Company at Montrale, 48^{th} edition, 1994, p. 2197-2199). Compared to other vasodilatory drugs (e.g. nitroglycerin, Prostaglandin E₁ etc.) misoprostol causes a stronger local vasodilation and as a result increase of the blood flow when it is used externally to the clitoris or/and to the vagina. Because of the local vasodilation tumescence of the clitoris, intense bleeding of the vagina and feeling of sexual desire are caused. Simultaneously, in women with anorgasmia of various causes, promote, after masturbation or sexual intercourse, the coming of orgasm. Upon application, equally strong topical vasodilation is exerted by misoprostol's hydrolysis product (misoprostol acid) which also constitutes the first misoprostol metabolite after its introduction to the body (see page 8, Fig. 2). Firstly because of the intense topical vasodilatory action of misoprostol and the corresponding free acid, the two pharmaceutical molecules reinforce the absorption of other vasoactive substances (e.g. alprostadil) resulting in the occurrence of synergic action.

Misoprostol can be dissolved in water and its compatibility with excipients provides the opportunity for the production of a variety of simple pharmaceutical dosage forms for external use, which are, at the same time, very well tolerated by the skin and the mucosa.

From the above-mentioned description it appears that the most serious advantage of the invention is the manner of administration of the drug (external, in combination with the lack of undesirable action in the suggested doses or/and the proposed pharmaceutical dosage forms), the relatively low cost and especially the most satisfactory result compared to conventional methods.

Amongst the probable forms of application, most advantageous is a composition in the gel form of relatively low viscosity which contains 0.3-0.9% w/v misoprostol in the methyl form of methylester and/or free acid, a complex forming means, as 1.6% w/v a cyclodextrin and substances suitable for the formation of a gel e.g. hydroxypropyl methylcellulose "3000" 2% w/v, propylene glycol 10% v/v and Water to 100 ml. The gel contains 3-9 mg of active substance per ml. Method of application: 0.1 (or more, depending on response) are applied to the clitoris or/and to the vagina.

Nine examples related to the pharmaceutical dosage forms and the ways of application of misoprostol are presented below:
1) 0.10 ml gel, relatively low viscosity containing 0.3-0.9% w/v misoprostol for applying to the clitoris or/and to the vagina.

| Synthesis: | |
|---|---|
| 1-1 Misoprostol | 0.3-0.9 g |
| Hydroxypropyl Methylcellulose "3000" | 2 g |
| Water purified to | 100 ml |
| 1-2 Misoprostol | 0.3-0.9 g |
| Sodium Carboxymethylcellulose | 2 g |
| Propylene Glycol | 25 ml |
| Water purified to | 100 ml |

2) 0.10 ml gel of relatively high viscosity, containing 0.30-0.90% w/v misoprostol for vaginal application.

| Synthesis: | |
|---|---|
| 2-1 Misoprostol | 0.30-0.90 g |
| Hydroxypropyl Methylcellulose "3000" | 4 g |
| Water purified to | 100 ml |
| 2-2 Misoprostol | 0.30-0.90 g |
| Sodium Carboxymethylcellulose | 4 g |
| Propylene Glycol | 25 ml |
| Water purified to | 100 ml |

3) 0.10 ml of aqueous solution of misoprostol containing 0.3-0.9% w/v for clitoral or/and vaginal application. The solution can also contain propylene glycol or glycerol in various amounts needed to increase the viscosity of the solution, e.g. 10%.
4) 0.10 ml of ointment or emulsion o/w containing 0.3-0.9% w/w misoprostol for clitoral or/and vaginal application, where misoprostol is found in the continuous (aqueous) phase.

| Synthesis: | |
|---|---|
| 4-1 Misoprostol | 0.3-0.9 g |
| Vanishing Cream to | 100 g |

(Although for the purposes of this example, Bepanthéne® Cream (Roche) was used as vanishing cream, various creams available in the market or described in National Pharmacopoeias can be used for the same purpose.)
5) Vaginal ovules of suitable dimensions, weighing about 300-900 mg and containing 0.04-0.20% w/w misoprostol for vaginal use.

| Synthesis: | |
|---|---|
| 5-1 Misoprostol | 0.3-0.9 g |
| Glycerol | 70 g |
| Gelatine | 20 g |
| Water purified to | 100 g |

6) 0.10 ml gel (or more, depending on response), according to examples (1-1) and (2-1) which contains moreover 1.6% w/v a-cyclodextrin.
7) 0.10 ml gel (or more, depending on response), according to example (6) which contains moreover 10 ml ethyl alcohol 96° and 0.5 mg/ml alprostadil.
Notes:
1) The incorporation of misoprostol in the above-mentioned bases took place at room temperature (20-25°C) and at a temperature not exceeding 40°C.
2) No significant changes in misoprostol activity was observed as a function of pH, we observant however an important reduction or/and neutralization of misoprostol action in the presence of Polysorbate "80".
3) The time of appearance of the result varies from 20-40 minutes. The timing of the appearance and the intensity of the result seems to be positively influenced by certain hydrating agents (e.g. Propylene Glycol, Glycerol) as well as by certain substances which increase the transcutaneous absorption by various mechanisms (e.g. Urea, Acid Citric).
4) High single doses of misoprostol (>1000 mcg to the clitoris or to the vagina) cause certain undesirable, systematic effects as shudder, feeling of hardship, excitement and diarrhoea. The presence of a-cycodextrin reduces these undesirable effects and allows the application of higher single doses without notable effect on the onset of action but with positive effect on the intensity of the result and prolonging of its duration.
5) The doses which are mentioned in the examples are only indicative since the intensity of the result depends, apart from the nature and the extent of sexual dysfunction, on other factors too, such as e.g. the degree of moisturisation of the underlying tissue, the physiological state of the skin or the mucosa etc.. As already mentioned, misoprostol is an extremely hydrophilic molecule compared with other prostaglandins of E₁ series (e.g. with alprostadil which can be dissolved in alcohol but its solubility in water is only 8000 mcg/100 ml at 35°C). This consists an important advantage:
   a) Because no use of organic solvents (e.g. ethyl alcohol) which usually irritate tissues and are thus unsuitable for application on the skin and especially the mucus is required.
   b) Because it allows the incorporation of the active substances in a very small amount of excipient, suitable for application on surfaces of limited size, as e.g. the clitoris.
6) Misoprostol hasn't been accused of carcinogenic or teratogenic effects, but because of the described irritation of the smooth uterine fibbers (Physicians Desc Reference, PDR, ed. Medical Economics Data, Production Company at Montrale 48^{th} edition, 1994, p. 2197-2199), misoprostol must not come in contact with the genital system of pregnant women.

## Claims

1. The use of misoprostol and/or its first metabolite, misoprostol acid, for the production of a drug applied topically to the clitoris and/or to the vagina and aimed at the therapy of sexual dysfunction in women due to vascular or other causes.

2. The use of misoprostol and/or its metabolite, misopmstol acid, according to claim 1, either as racemic mixtures or in the form of one of the stereoisomers of which they consist: [(±)-R form & (±)-S form].

3. The use of misoprostol and misoprostol acid according to claims 1 and 2 in the form of various galenic preparations (solutions, ointments, vaginal sticks, systems of controlled transdermal absorption) which facilitate the application and precise administration of the right doses for the achievement of the desired therapeutical aim as described in claim 1.

4. The use of misoprostol and misoprostol acid according to claims 1, 2 and 3 in combination with other vasodilatory drugs as alprostadil for the appearance of synergistic action, as well as "penetration enhancers" used normally to increase absorption of drugs through the skin or the mucosa.

5. Use of misoprostol and misoprostol acid according to claims 1, 2 and 3 in combination with a-cyclodextrin or other substances which impede or retard the appearance of undesired effects of the drug or prolong their pharmaceutical action.

6. Use of misoprostol and/or misoprostol acid for the manufacture of a medicament for sexual dysfunction in women.

## Patentansprüche

1. Verwendung von Misoprostol und/oder seines Erstmetaboliten, der Misoprostolsäure, zur Herstellung eines Arzneimittels, das topisch auf die Klitoris und/oder auf die Vagina aufgebracht wird und zur Therapie der sexuellen Dysfunktion bei Frauen aufgrund vaskulärer oder anderer Ursachen vorgesehen ist.

2. Verwendung von Misoprostol und/oder seinem Metaboliten, der Misoprostolsäure, gemäß Anspruch 1, entweder als razemische Gemische oder in Form eines der Stereoisomeren, aus denen sie bestehen: [(±)-R Form & (±)-S Form].

3. Verwendung von Misoprostol und Misoprostolsäure nach Anspruch 1 und 2 in Form verschiedener galenischer Zubereitungen (Lösungen, Salben, Vaginalstäbchen, Systeme zur kontrollierten transdermalen Absorption), die die Anwendung und präzise Verabreichung der richtigen Dosen zum Erreichen des erwünschten therapeutischen Zweckes wie in Anspruch 1 beschrieben, erleichtern.

4. Verwendung von Misoprostol und Misoprostolsäure nach Anspruch 1, 2 und 3 in Kombination mit anderen vasodilatatorischen Arzneistoffen wie Alprostadil zum Erreichen einer synergistischen Wirkung ebenso wie mit "Penetrationsverbesserern", die normalerweise dazu verwendet werden, die Absorption von Arzneimitteln durch die Haut oder die Schleimhaut zu erhöhen.

5. Verwendung von Misoprostol und Misoprostolsäure nach den Ansprüchen 1, 2 und 3 in Kombination mit a-Cyclodextrin und anderen Substanzen, die das Auftreten unerwünschter Wirkungen des Arzneimittels verhindern oder verlangsamen oder seine pharmazeutische Wirkung verlängern,

6. Verwendung von Misoprostol und/oder Misoprostolsäure zur Herstellung eines Medikamentes gegen die sexuelle Dysfunktion bei Frauen.

## Revendications

1. Utilisation de misoprostol et/ou de son premier métabolite, l'acide misoprostolique, pour la production d'un médicament appliqué par voie topique sur le clitoris et/ou le vagin et destiné au traitement du dysfonctionnement sexuel chez la femme dû à des causes vasculaires ou autres.

2. Utilisation de misoprostol et/ou de son métabolite, l'acide misoprostolique, selon la revendication 1, soit en tant que mélanges racémiques, soit sous la forme d'un des stéréoisomères en lesquels ils consistent : [forme (±)-R et forme (±)-S].

3. Utilisation de misoprostol et d'acide misoprostolique selon les revendications 1 et 2, sous la forme de diverses préparations galéniques (solutions, pommades, sticks vaginaux, systèmes d'absorption transdermique contrôlée) qui facilitent l'application et une administration précise des doses adéquates pour obtenir le but thérapeutique souhaité comme décrit dans la revendication 1.

4. Utilisation de misoprostol et d'acide misoprostolique selon les revendications 1, 2 et 3 en association avec d'autres médicaments vasodilatateurs comme l'alprostadil pour l'apparition d'une action synergique, ainsi que des « agents renforçant la pénétration » utilisés normalement pour accroître l'absorption de médicaments à travers la peau ou les muqueuses.

5. Utilisation de misoprostol et d'acide misoprostolique selon les revendications 1, 2 et 3 en association avec l'a-cyclodextrine ou d'autres substances qui empêchent ou retardent l'apparition d'effets indésirables du médicament ou prolongent son action pharmaceutique.

6. Utilisation de misoprostol et/ou d'acide misoprostolique pour la fabrication d'un médicament destiné à traiter le dysfonctionnement sexuel chez la femme.
